**Europäisches Patentamt**

**European Patent Office**

**Office européen des brevets**

(11) Veröffentlichungsnummer: **0 128 566**
**A2**

# EUROPÄISCHE PATENTANMELDUNG

(21) Anmeldenummer: **84106605.3**

(22) Anmeldetag: **08.06.84**

(51) Int. Cl.³: **C 12 N 13/00**
**// C12N15/00**

(30) Priorität: **11.06.83 DE 3321239**

(43) Veröffentlichungstag der Anmeldung: **19.12.84**
**Patentblatt 84/51**

(84) Benannte Vertragsstaaten: **CH FR GB IT LI**

(71) Anmelder: **Kernforschungsanlage Jülich Gesellschaft mit beschränkter Haftung, Postfach 1913, D-5170 Jülich (DE)**

(72) Erfinder: **Zimmermann, Ulrich, Prof., Im Geyberg 21, D-5165 Hürtgenwald-Gey (DE)**
Erfinder: **Arnold, William Michael, Adalbertstrasse 116-118, D-5100 Aachen (DE)**
Erfinder: **Büchner, Karl-Heinz, Schützenstrasse 19, D-5170 Jülich (DE)**
Erfinder: **Matschke, Christian, Wirthstrasse 63, D-5110 Alsdorf (DE)**

(54) **Kammer zur Behandlung von Zellen im elektrischen Feld.**

(57) Die Erfindung bezieht sich auf eine Kammer für die Behandlung von Zellen im elektrischen Feld, bei der ein von einer Wandung aus elektrisch nichtleitendem Material begrenzter Raum zur Aufnahme der die Zellen enthaltenden Suspension vorgesehen ist. In den Raum ragen wenigstens zwei Elektroden derart hinein, dass ein zwischen den Elektroden liegender Bereich gebildet wird, in welchem die Zellen einem zwischen den Elektroden ausgebildeten elektrischen Feld ausgesetzt sind. Erfindungsgemäss ist der Raum allseitig hermetisch verschlossen. Wenigstens eine Stelle der Wandung ist dabei so beschaffen, dass sie mittels einer Nadel, insbesondere der Kanüle einer Injektionsspritze, perforierbar ist. Bei einer Ausführungsform der Kammer besteht die Wandung teilweise aus einer perforierbaren Folie.

EP 0 128 566 A2

0128566

Kernforschungsanlage Jülich
Gesellschaft mit beschränkter Haftung

Kammer zur Behandlung von Zellen im elektrischen
Feld

Die Erfindung bezieht sich auf eine Kammer für die Behandlung von Zellen im elektrischen Feld, bei der ein von einer Wandung aus elektrisch nichtleitendem Material begrenzter Raum zur Aufnahme der die Zellen enthaltenden Suspension vorgesehen ist, in den wenigstens zwei Elektroden derart hineinragen, daß ein zwischen den Elektroden liegender, durch diese begrenzter Bereich gebildet wird, in welchem die Zellen einem zwischen den Elektroden ausgebildeten elektrischen Feld ausgesetzt sind.

Aus der DE-PS 24 05 119 ist ein Verfahren zur Behandlung von Zellen im elektrischen Feld bekannt, bei dem die Membran von Zellen durch Anwendung eines elektrischen Feldes, dessen Stärke $10^3$ bis $10^5$ V/cm beträgt, durchbrochen wird. Die dabei bewirkte Permeabilitätserhöhung der Zellmembran macht es möglich, Stoffe durch die Membran auszutauschen, ohne daß die Zellen in ihrer Lebensfähigkeit beeinträchtigt sind. Denn die Erhöhung der Permeabilität ist nach dem Austausch der Stoffe in einem einfachen Verfahrensschritt reversibel. Auf diese Weise ist es beispielsweise auch möglich, Gene oder Enzyme in die Zellen einzulagern.

ha

- 2 -

Das aus der vorgenannten Patentschrift bekannte
Verfahren der Erhöhung der Permeabilität der
Zellmembran ist auch zur Fusion von Zellen
einsetzbar.

Zwei Zellen in einer Suspension sollten, wenn
sie sich berühren und ein enger Kontakt zwischen
den Membranen beider Zellen entsteht, miteinander verschmelzen, da die Bausteine in der
Membran beweglich sind. Eine derartige spontane
Verschmelzung (Fusion) von Zellen wird unter
natürlichen Bedingungen jedoch nicht oder nur
äußerst selten beobachtet. Eine bekannte Ausnahme stellt die Befruchtung einer Eizelle
durch eine Samenzelle bei der sexuellen Fortpflanzung dar. Die spontane Fusion wird durch
die negative Ladung der Phospholipide und anderer Membrankomponenten behindert. Sie führt
zur Abstoßung der Zellen, wenn sie sich auf
einen geringen Abstand genähert haben. Zellverschmelzung erfordert aber, daß die beiden
Membranen sich bis auf einen Abstand von weniger
als $10^{-9}$ m nähern können.

Die mit technischen Mitteln, also auf künstlichem
Wege durchgeführte Fusion von Zellen ist in
einem weiten Anwendungsbereich einsetzbar.
So ist es für die biologisch-medizinische Forschung von großem Interesse, eine große Zahl
von Zellen miteinander zu verschmelzen. Bei
geeigneter Größe der durch Fusion mehrerer
oder gegebenenfalls vieler Zellen - beispielsweise
1000 bis 10000 Blutkörperchen -

- 3 -

- 3 -

entstandenen großen Zellen lassen sich dann Mikrolektroden, Mikrodruckmeßsonden und andere Sensoren ohne irreversible Zerstörung der Membran in die große Zelle einführen. Die Technik, über die Sensoren direkt eine Reihe von Zellen und Membranfunktionen zu erfassen, ist dabei für die klinische Diagnostik, z.B. bei der Früherkennung von Erkrankungen sowie generell für die Grundlagenforschung von Bedeutung.

Die Technik der Fusion von Zellen kann außerdem eingesetzt werden für die Bildung von Hybridzellen durch Verschmelzung von zwei Zellen unterschiedlicher Herkunft, die evolutionsmäßig nicht zu weit voneinander entfernt stehen sollen. Dabei können Zellhybride aus Pflanzenzellen, aus denen wieder ganze Pflanzen gezüchtet werden können oder Zellhybride aus tierischen Zellen, über die monoklonale Antikörper, z.B. gegen Tumore und Leukämie, gewonnen werden können, gebildet werden. Als Beispiel sei genannt die Verschmelzung einer Lymphozytenzelle mit einer Myelomzelle, die besonders aus medizinischer und pharmazeutischer Sicht von großem Interesse ist. Bestimmte Lymphozyten bilden gegen Fremdstoffe im Organismus Antikörper, z.B. gegen ein Fremdeiweiß, das in die Blutbahn injiziert worden ist. Isoliert man die Lymphozyten und fusioniert sie mit einer Tumorzelle, wie der Myelomzelle, so besteht die Chance, daß sich eine sogenannte Hybridomzelle bildet, die die Eigenschaft beider Elternzellen

- 4 -

- 4 -

besitzt. Diese Zelle produziert Antikörper,
und zwar spezifisch nur gegen den betreffenden
Fremdstoff (sogenannte monoklonale Antikörper).
Sie ist unsterblich und läßt sich im Gegensatz
zu einer normalen ausdifferenzierten Zelle,
wie dem Lymphozyten, permanent in Nährmedien
vermehren.

Ein Verfahren zur Fusion von Zellen der eingangs
bezeichneten Art ist aus Biochimica et Biophysica
Acta, 694 (1982), 227 - 277 (Electric Field-Mediated
Fusion And Related Electrical Phenomena, U. Zimmermann) bekannt. Bei diesem bekannten Verfahren
- dessen Ablauf unter dem Mikroskop beobachtet
werden kann - wird der Membrankontakt zwischen
wenigstens zwei Zellen durch Anlegen eines alternierenden, schwach homogenen Feldes erzeugt.
Durch das elektrische Feld werden, bedingt durch
Polarisationsprozesse in der Zelle, Dipole erzeugt,
die sich gegenseitig anziehen, wenn sich die
Zellen während ihrer Wanderung im elektrischen
Feld einander nähern (sogenannte Dielektrophorese).
Nach der Bildung der Zellenreihe werden die
Störungen in der Membranstruktur zwischen benachbarten Zellen durch einen elektrischen Durchbruchpuls ausgelöst (J. Membrane Biol. 67, 165 - 182
(1982), Electric Field-Induced Cell-to- Cell Fusion,
U. Zimmermann and J. Vienken). Dabei werden
- nach den bisherigen Modellvorstellungen -
Löcher in der Membrankontaktzone benachbarter
Zellen erzeugt, die zu einem zytoplastischen
Kontinuum zwischen den beiden Zellen und zur

- 5 -

Brückenbildung von Lipiden zwischen den Membranen
der benachbarten Zellen führen. Die Lipidmoleküle
ordnen sich nicht mehr in ihre ursprüngliche
Membran ein. Sobald sich eine Brücke gebildet
hat, kommt es aus energetischen Gründen zur
Abrundung des entstandenen Gebildes, das aus
den über die Lipidbrücken miteinander verbundenen
Zellen besteht.

Zur Durchführung dieser bekannten Verfahren
wird eine Kammer der eingangs bezeichneten Art
eingesetzt. Die Elektroden der Kammer sind dabei
zur Bildung der Zellenreihe an eine Einrichtung
zur Erzeugung eines alternierenden elektrischen
Feldes und zur Erzeugung des elektrischen Durchbruches an eine Einrichtung zur Erzeugung elektrischer Spannungspulse angeschlossen.

Es ist Aufgabe der Erfindung, eine Kammer zu
schaffen, die möglichst einfach zu handhaben
ist.

Diese Aufgabe wird gemäß der Erfindung dadurch
gelöst, daß der Raum der Kammer, der zur Aufnahme
der die Zellen enthaltenden Suspension vorgesehen ist, allseitig hermetisch verschlossen
ist und wenigstens eine Stelle der Wandung so
beschaffen ist, daß sie mittels einer Nadel,
insbesondere der Kanüle einer Injektionsspritze,
perforierbar ist.

Bei der Herstellung der Kammer wird deren Raum zweckmäßigerweise unter keimfreier Atmosphäre verschlossen. Die Kammer kann daher, ohne daß besondere Vorkehrungen getroffen werden müßten, direkt zum Einsatz kommen, wobei lediglich die Elektroden an die elektrische Einrichtung anzuschließen sind. Der Kammerraum wird sodann mit der Zellensuspension gefüllt, indem die perforierbare Stelle der Wandung mit einer feinen Nadel durchlöchert und über eine zweite Perforation Zellensuspension in den Raum eingespritzt wird. Das im Kammerraum befindliche keimfreie Gas wird dabei durch die erste Perforation aus dem Raum gedrückt.

Eine zweckmäßige Ausführungsform der Kammer gemäß der Erfindung besteht darin, daß die Wandung teilweise aus einer perforierbaren Folie, beispielsweise einer Kunststoffolie, besteht. Für den als Folie ausgebildeten Teil der Wandung wird dabei zweckmäßigerweise der obere, leicht zugängliche Teil der Wandung vorgesehen.

Ein Ausführungsbeispiel der Kammer gemäß der Erfindung ist in der Zeichnung schematisch dargestellt und wird im folgenden näher erläutert:

Es zeigen

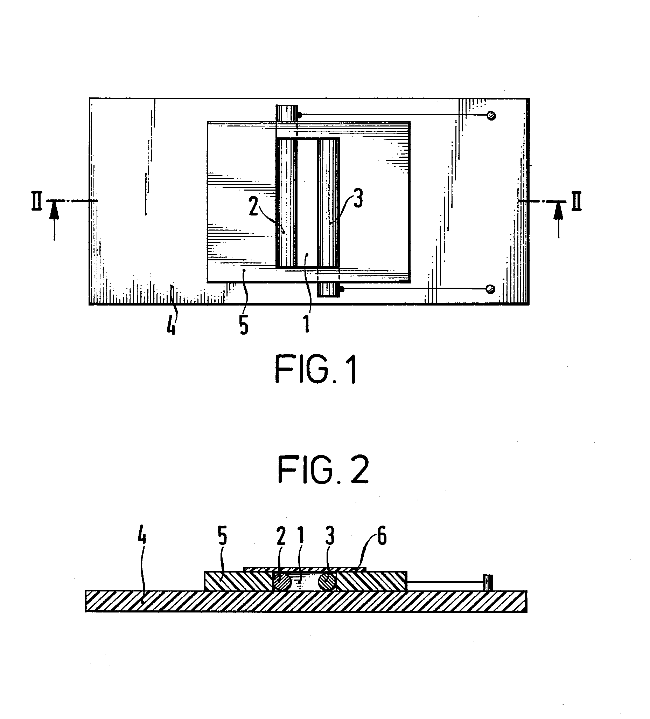

Figur 1    eine Aufsicht auf die Kammer,
Figur 2    einen Längsschnitt durch die Kammer
           gemäß Figur 1 entlang der Linie A-B.

Wie aus der Zeichnung ersichtlich ist, weist die Kammer einen Raum 1 auf, in den zwei Elektroden,

- 7 -

2 und 3, hineinragen. Die Wandung des Raumes 1 wird von einer Grundplatte 4, einem die seitlichen Wände bildenden Seitenteil 5 und der Folie 6 gebildet. Der Abstand zwischen den Elektroden liegt im allgemeinen - je nach Art und Größe der zu behandelnden Zellen - im Bereich von 5 µm bis 1000 µm.

Grundplatte 4 und Seitenteil 5 bestehen aus Polymethacrylat, die Folie ist aus Acetylzellulose.

-1-

0128566

Kernforschungsanlage Jülich
Gesellschaft mit beschränkter Haftung

P a t e n t a n s p r ü c h e

1. Kammer für die Behandlung von Zellen im elektrischen Feld, bei der ein von einer Wandung aus elektrisch nicht leitendem Material begrenzter Raum zur Aufnahme der die Zellen enthaltenden Suspension vorgesehen ist, in den wenigstens zwei Elektroden derart hineinragen, daß ein zwischen den Elektroden liegender, durch diese begrenzter Bereich gebildet wird, in welchem die Zellen einem zwischen den Elektroden ausgebildeten elektrischen Feld ausgesetzt sind, d a d u r c h   g e k e n n - z e i c h n e t , daß der Raum (1) allseitig hermetisch verschlossen ist und wenigstens eine Stelle (6) der Wandung so beschaffen ist, daß sie mittels einer Nadel, insbesondere der Kanüle einer Injektionsspritze, perforierbar ist.

2. Kammer nach Anspruch 1, d a d u r c h   g e - k e n n z e i c h n e t , daß die Wandung teilweise aus einer perforierbaren Folie (6) besteht.

PT 1.680

ba/ha

M 08·06·84
0128566

FIG.1

FIG.2